# EUROPEAN PATENT SPECIFICATION

(11) **EP 1 931 996 B1**
(45) Date of publication and mention of the grant of the patent: **20.11.2013**
(21) Application number: 07751053.5
(22) Date of filing: 16.02.2007
(51) Int. Cl.: G01N 33/574

(54) **FREE NGAL AS A BIOMARKER FOR CANCER**
FREIES NGAL ALS BIOMARKER FÜR KREBS
NGAL LIBRE EN TANT QUE BIOMARQUEUR DE CANCER

(30) Priority: 17.02.2006 US 774823 P
(43) Date of publication of application: 18.06.2008
(60) Divisional application: 11156071.0
(73) Proprietor: Children's Medical Center Corporation, Boston, MA 02115 (US)
(72) Inventor: MOSES, Marsha, A., Brookline, MA 02145 (US); JANG, Jiang, Melrose, MA 02176 (US)
(74) Representative: Williams, Gareth Owen
(86) International application number: PCT/US2007/004265
(87) International publication number: WO 2007/098102

(56) References cited:
- WO-A-02/31507
- WO-A-96/32647
- WO-A-02/071928
- WO-A-2004/088276
- FERNÁNDEZ CECILIA A ET AL: "The matrix metalloproteinase-9/neutrophil gelatinase-associated lipocalin complex plays a role in breast tumor growth and is present in the urine of breast cancer patients." CLINICAL CANCER RESEARCH : AN OFFICIAL JOURNAL OF THE AMERICAN ASSOCIATION FOR CANCER RESEARCH 1 AUG 2005, vol. 11, no. 15, 1 August 2005 (2005-08-01), pages 5390-5395, XP002440683 ISSN: 1078-0432
- ANONYMOUS: "Quantikine - Human Lipocalin-2/NGAL Immunoassay" CATALOG NUMBER DLCN20, [Online] XP002440684 Retrieved from the Internet: URL:http://www.rndsystems.com/pdf/DLCN20.p df> [retrieved on 2006-02-03]
- KJELDSEN L ET AL: "Characterization of two ELISAs for NGAL, a newly described lipocalin in human neutrophils" JOURNAL OF IMMUNOLOGICAL METHODS, ELSEVIER SCIENCE PUBLISHERS B.V.,AMSTERDAM, NL, vol. 198, no. 2, 13 November 1996 (1996-11-13), pages 155-164, XP004071802 ISSN: 0022-1759
- FRIEDL A ET AL: "Neutrophil gelatinase-associated lipocalin in normal and neoplastic human tissues. Cell type-specific pattern of expression." THE HISTOCHEMICAL JOURNAL JUL 1999, vol. 31, no. 7, July 1999 (1999-07), pages 433-441, XP002440685 ISSN: 0018-2214
- YAN LI ET AL: "The high molecular weight urinary matrix metalloproteinase (MMP) activity is a complex of gelatinase B/MMP-9 and neutrophil gelatinase-associated lipocalin (NGAL). Modulation of MMP-9 activity by NGAL.MODULATION OF MMP-9 ACTIVITY BY NGAL" JOURNAL OF BIOLOGICAL CHEMISTRY, AMERICAN SOCIETY OF BIOLOCHEMICAL BIOLOGISTS, BIRMINGHAM,, US, vol. 276, no. 40, 5 October 2001 (2001-10-05), pages 37258-37265, XP002226670 ISSN: 0021-9258

## Description

### FIELD OF THE INVENTION

The invention relates to non-invasive methods for the diagnosis and prognosis of cancer, *e.g*., cancers of epithelial origin including breast and ovarian cancer, by determining free NGAL levels in urine samples obtained from a patient.

### BACKGROUND OF THE INVENTION

One of the most important factors in the survival of cancer is early stage detection. Clinical assays that detect the early events of cancer offer an opportunity to intervene and prevent cancer progression. With the development of gene profiling and proteomics, there has been significant progress in the identification of molecular markers or "biomarkers" that can be used to diagnose and prognose specific cancers. For example, in the case of prostate cancer, PSA (prostate specific antigen) can be detected in the blood, and is indicative of the presence of prostate cancer. Thus, the blood of men at risk for prostate cancer can be quickly, easily, and safely screened for elevated PSA levels.

Despite progress in the field of cancer detection, there still remains a need in the art for the identification of new biomarkers for a variety of cancers that can be easily used in clinical applications, particularly non-invasive methods of cancer screening. Urinalysis presents probably the most patient-friendly option (*e.g*., it does not require phlebotomy) and is widely used by health care providers, but to date there appear to be few options available for diagnosing cancer in this manner.

Polymorphic epithelial mucin (MUC1) is a transmembrane protein overexpressed in ovarian cancer, and the tumor expressed MUC1 protein is often cleaved into the circulation, where it is detectable as the tumor marker, CA 15-3; *see, e.g.,* Bon et al. Clin. Chem. 43:585(1997). However, many patients have tumors that do not express MUC1. Serum CA125 levels have also been reported to be associated with ovarian cancer (Skakes, *Cancer* 76:2004(1995)); but assessment of these levels are not absolute indicators of disease. Although roughly 85% of women with clinically apparent ovarian cancer have increased levels of CA125, CA125 is also increased during the first trimester of pregnancy, during menstruation, in the presence of non-cancerous illnesses, and in cancers of other sites.

The expression of the gene for neutrophil gelatinase associated lipocalin (NGAL; lipocalin-2) has been identified as being up-regulated in ovarian, colon, lung, and uterine cancer tumor tissue (*see, .e.g.* WO02/102235, WO02/071928 and US2004/0005563) using gene expression analysis.

Fernandez et al, Clinical Cancer Research 2005; 11 (15), page 5390 to 5395 describes the detection of MMP9/NGAL complexes in the urine of breast cancer patients, and suggests that the urinary detection of these complexes may be useful in predicting the disease status of breast cancer patients.

WO02/3150 describes methods for diagnosing and prognosing cancers by the identification of high molecular weight enzyme complexes comprising MMPs. The detection of MMP9/NGAL complexes in urine of cancer patients is described.

WO02/07192 describes various markers associated with ovarian cancer, identified by transcriptional profiling of mRNA from ovarian cancer tumours. The markers include NGAL. The publication suggests that detecting levels of expression of the markers in patients may be useful in determining whether a patient has ovarian cancer or has an increased risk of developing cancer.

### SUMMARY OF THE INVENTION

The present invention provides a method for determining if an individual is at risk of developing, or has developed, cancer of epithelial origin, comprising determining a level of free NGAL in a test urine sample obtained from an individual and comparing the level of free NGAL in the test urine sample with a control level of free NGAL, in accordance with the method of claim 1.

The invention also provides a method for staging a cancer of epithelial origin comprising determining a level of free NGAL in a test urine sample, in accordance with independent claim 12.

Also provided is a method for monitoring cancer or risk of developing cancer of epithelial origin comprising determining levels of free NGAL in first and second urine samples in accordance with independent claim 15.

Also provided is a method of monitoring for cancer recurrence in a post cancer treatment patient, wherein the cancer is of epithelial origin, in accordance with independent claim 18.

Other features of the invention are described in the dependent claims.

Identifying biomarkers is particularly relevant to improving diagnosis, prognosis, and treatment of cancers, *e.g*., cancers of epithelial origin including breast and ovarian cancer, and as such there is a need in the art for biomarkers that can be quickly, easily and safely detected. The invention described herein utilizes a biomarker, free urinary neutrophil gelatinase associated lipocalin (NGAL), to diagnose, to stage, or to monitor the progression or treatment of a subject with cancer of epithelial origin, in particular, an invasive, potentially metastatic stage of the disease. The prior work in this field has not shown or suggested this as a reliable, non-invasive way to detect cancers, *e.g*., of epithelial origin including breast cancer or ovarian cancer.

As described herein, uncomplexed or "free" NGAL has been found to be present at increased levels in the urine of individuals with atypical ductal hyperplasia (ADH), a major risk factor for future breast cancer development; in individuals that have ovarian cancer; and in individuals that have breast cancer, both invasive and non-invasive. Accordingly, the invention encompasses measuring uncomplexed urinary NGAL as a primary screen to determine if an individual is either at risk of developing, or has developed, a cancer of epithelial origin such as ovarian, prostate, or breast cancer; and other cancers such as basal cell carcinoma, renal cell carcinoma, adenocarcinoma, gastrointestinal cancer, lip, mouth, esophageal, small bowel, stomach, colon, liver, bladder, pancreatic, cervical, lung, skin, and kidney cancer. Methods for monitoring levels of urinary NGAL as a marker for therapeutic efficacy are also disclosed.

In an embodiment, the invention includes methods for determining if an individual is at risk of developing, or has developed, cancer of epithelial origin. A level of free NGAL in a test urine sample obtained from an individual is determined, and this level is compared with a control level of free NGAL. A level of free NGAL in the test sample higher than a control level of free NGAL indicates that the individual is either at increased risk for developing cancer or has cancer of epithelial origin. In another embodiment, the individual is a post-cancer treatment patient, and when a level of free NGAL in the test sample is higher than a control level of free NGAL, the post-cancer treatment patient is referred for treatment of recurrence of the cancer. The cancer is a cancer of epithelial origin, *e.g*., breast cancer, basal cell carcinoma, adenocarcinoma, gastrointestinal cancer, lip cancer, mouth cancer, esophageal cancer, small bowel cancer, stomach cancer, colon cancer, liver cancer, bladder cancer, pancreas cancer, ovary cancer, cervical cancer, lung cancer, skin cancer, kidney cancer, prostate cancer, or renal cell carcinoma. In another embodiment, the level of free NGAL detected is monomeric NGAL. In another embodiment, the level of free NGAL detected includes monomeric NGAL. In another embodiment, an antibody-based binding moiety (*e.g*. a monoclonal antibody) which preferentially binds to free NGAL to form an antibody-NGAL complex is used, and the presence of the antibody-NGAL complex is detected to measure the level of free NGAL present. The antibody-based binding moiety may be labelled with a detectable label such as a radioactive label, a hapten label, fluorescent label, or an enzymatic label.

In another embodiment, the invention includes methods for staging a cancer of epithelial origin. A level of free NGAL in a test urine sample obtained from an individual is determined, and this level is compared with a control level of free NGAL. A level of free NGAL, *e.g*., monomeric NGAL, in the test sample higher than a control level of free NGAL indicates that the individual is either at increased risk for developing or has malignant or metastatic cancer. The control level of free NGAL may be associated with a healthy individual.

Methods for directing treatment of a subject are described. A subject is tested for levels of free NGAL in a urine sample obtained from the subject, a clinician reviews the results, and if the urine has a higher level of free NGAL than the control level of free NGAL, the clinician directs the subject to be further tested for cancer. The test may be performed in the same country where the subject resides, or in another country, and the results are made available, for example via a Web site, or are transmitted to the clinician.

Methods for monitoring a cancer or risk of cancer of epithelial origin are also disclosed. A level of free NGAL in a first test urine sample obtained from an individual to obtain an initial level of free NGAL is determined, and the level of free NGAL in the test urine sample is compared with a level of free NGAL in a second urine sample obtained from the individual to obtain a second level of free NGAL. Subsequent urine collection and comparisons at selected intervals, *i.e*., to collect a set of data points, is done, and the data points are reviewed to determine if the measured levels of free NGAL in the test samples versus the measured levels of free NGAL in the control sample trend upward, the upward trend indicating that a cancer is further developing or metastasizing in the individual; *i.e*., if the second level is greater than the first level, the individual may be deemed at greater risk of having or developing cancer, or is at greater risk of having or developing a metastasizing cancer. In another embodiment, the individual is a post-cancer treatment patient, and when the second level is greater than the first level, the post-cancer treatment patient is referred for treatment of recurrence of the cancer

Kits for detecting free NGAL in a urine sample are also disclosed. The kits include a container for holding a urine sample, and at least one antibody that preferentially binds to free NGAL; and directions for use. The kit may alternatively include at least two antibodies that preferentially bind to NGAL, one of the antibodies being immobilized on a solid phase and the other of the antibodies being detectably labeled.

NGAL is found in urine as both free NGAL, *i.e*., not bound to matrix metalloproteinase 9 (MMP-9) and as complexed NGAL, i.e. NGAL/MMP-9 complex. In the methods described herein, free NGAL is measured.

In one embodiment, a method determining if an individual is at risk of developing, or has developed a cancer of epithelial origin including breast and ovarian cancer, is provided. The method comprises measuring levels of free and complexed NGAL present in urine of a test sample and comparing the levels obtained in the test sample with a control sample, wherein higher levels of NGAL in the urine of the test sample indicates that the individual is either at increased risk for developing cancer or already has a cancer. A positive result (higher levels) therefore indicates that the individual should be further tested for cancer and monitored accordingly. Preferably, the level of free NGAL is 1.5 fold, more preferably 2 fold or greater, more than that of the control. This level of increase can also be detected against a control level.

The invention is advantageously used for risk assessment or early detection of cancer of epithelial origin including breast and ovarian cancer. For example, a subject can be screened by a physician during their annual physicals. A positive test result, wherein the levels of free NGAL are higher than that of the control, would warrant further diagnostic evaluation to determine whether or not the individual has a cancer precursor lesion or cancer.

As noted above, the invention may also be used to assess the level of urinary free NGAL present in multiple test samples obtained from the same patient, where a progressive increase in the amount of free NGAL over time indicates an increased aggressiveness (*e.g*., metastatic potential) of the cancer of epithelial origin. As such, the NGAL levels serve as a predictor of disease status and stage.

As described herein, NGAL levels are assessed at one or more intervals to monitor the therapeutic efficacy of a treatment regime designed to treat cancer, *e.g*., cancer of epithelial origin including breast and ovarian cancer, patient.

In one aspect of the invention, NGAL levels present in a test urine sample are measured by contacting the test sample, or preparation thereof, with an antibody-based binding moiety that preferentially binds specifically to (free) NGAL protein, or to a portion thereof. By "preferentially" is meant that the antibody binds to free NGAL, rather than NGAL that may be complexed (*e.g*., to MMP-9). In an embodiment, the free NGAL is monomeric NGAL. The antibody-based binding moiety forms a complex with NGAL that can be detected, thereby allowing the levels of NGAL to be measured. To distinguish between free NGAL and complexed NGAL, an antibody-based binding moiety that preferentially interacts with free NGAL can be used, *e.g*., in an ELISA. Such antibody-binding moieties may be raised against an epitope inside the MMP-9/NGAL binding region.

Antibody-based immunoassays are the preferred means for measuring levels of NGAL protein. However, any means known to those skilled in art can be used to assess NGAL levels. For example, in some embodiments NGAL expression levels are assayed by mass spectrometry, including SELDI mass spectrometry.

### BRIEF DESCRIPTION OF THE DRAWINGS

The accompanying drawings, which are incorporated in and constitute a part of this specification, illustrate embodiments of the invention and, together with the description, serve to explain the objects, advantages, and principles of the invention.

**Figures 1A and 1B** show NGAL transcript expression levels in ovarian cancer cell lines. Expression levels were determined using real-time PCR. **Figure 1A** shows a transcript separated on agarose as compared to GAPDH control transcript. **Figure 1B** is a graph depicting the ratio of NGAL/GAPDH on the Y-axis. HOSE is a normal ovarian cell line; OVCAR-3, OVCAR-5 and SKOV-3 are ovarian cancer cell lines.

**Figure 2** is a graph illustrating the amount of NGAL protein secretion in conditioned medium from ovarian cancer cell lines, OVCAR-3, OVCAR-5, and SKOV-3, as determined by Borregaard ELISA.

**Figures 3A and 3B** show NGAL transcript expression levels in breast cancer cell lines. Expression levels were determined using real-time PCR. **Figure 3A** shows a transcript separated on agarose as compared to GAPDH control transcript. **Figure 3B** is a graph depicting the ratio of NGAL/GAPDH on the Y-axis. T-47D and MCF-7 are "organ confined" breast cancer cell lines, while MDA-MB-231 is a "highly metastatic cell line".

**Figure 4** is a graph illustrating the amount of NGAL protein secretion in conditioned medium from breast cancer cell lines, T-47D, MCF-7 and MDA-MB-231, as determined by Borregaard ELISA.

**Figures 5A and 5B** show the levels of NGAL protein in urine samples from patients with either no disease, benign ovarian disease, and malignant ovarian cancer. Figure 5A shows a Western blot analysis of urine samples using anti-NGAL antibody. **Figure 5B** shows quantitative analysis of Western blots as described in **Figure 5A****.** Blots were quantitated using densitometry and levels are represented in arbitrary densitometric units.

**Figure 6** is the amino acid sequence of human NGAL (SEQ ID NO:1).

**Figure 7** is a graph of NGAL protein expression in ovarian cancer urine samples compared to healthy control samples as determined by Borregaard ELISA.

**Figure 8** is a graph of NGAL protein expression in breast cancer urine samples compared to healthy control samples as determined by Borregaard ELISA.

**Figure 9** is a graph of NGAL protein expression in individuals with atypical ductal hyperplasia (ADH) or lobular carcinoma *in situ* (LCIS), a major risk factor for future breast cancer development, as compared to levels seen in healthy individuals. R & D ELISA was used; see Example 1.

**Figure 10** is a graph illustrating the amount of migrated cells per field (y-axis) in a migration assay of breast cancer cells: MCF-7 cells, which express little NGAL, and two cell line clones that overexpress NGAL, N1 and N2.

**Figure 11** is a graph illustrating the amount of cell invasion in a tumor invasion assay of breast cancer cells: MCF-7 cells, which express little NGAL, and two cell line clones that overexpress NGAL, N 1 and N2.

**Figure 12** is a graph of NGAL protein expression in individuals with atypical ductal hyperplasia (ADH), a major risk factor for future breast cancer development, with ductal carcinoma in situ (DCIS), the most common type of noninvasive breast cancer, and with invasive breast cancer (IBC) as compared to levels seen in healthy individuals. R&D ELISA was used.

**Figure 13** is a graph showing the amount of free NGAL monomer present in ovarian cancer urine samples, from patients having normal, benign and malignant tumors. The malignant tumor samples evidence a higher free NGAL level than the benign sample population. These studies were carried out with an NGAL ELISA kit #DLCN20 (available from R&D Systems, Minneapolis, MN USA.).

### DETAILED DESCRIPTION OF THE INVENTION

### Definitions

"Free NGAL" is used interchangeably with the term "uncomplexed NGAL" and refers to NGAL that is not complexed with a matrix metalloproteinase, *e.g*., MMP-9.

"Cancers of epithelial origin" include cancers that arise from epithelial cells which include, but are not limited to, breast cancer, basal cell carcinoma, adenocarcinoma, gastrointestinal cancer, lip cancer, mouth cancer, esophageal cancer, small bowel cancer and stomach cancer, colon cancer, liver cancer, bladder cancer, pancreatic cancer, ovarian cancer, cervical cancer, lung cancer, breast cancer and skin cancer, such as squamous cell and basal cell cancers, prostate cancer, renal cell carcinoma, and other known cancers that effect epithelial cells throughout the body. A cancer of epithelial origin may be a hormone-dependent cancer, *e.g*., kidney, breast, endometrial, ovarian or prostate cancer.

"Control sample" includes urine sample(s) obtained from a "normal" or "healthy" individual(s) believed not to have cancer. Controls may be selected using methods that are well known in the art. Once a level has become well established for a control population, array results from test urine samples can be directly compared with the known levels. Preferably the control sample is an age and sex.matched control urine sample.

A "control level" is a level of analyte against which a tested level of the analyte is compared. The control level can be an empirically-determined mean or median analyte level from a group of individuals believed not to have the disease of interest (*e.g*., cancer) or having a particular stage of the disease of interest, *e.g*., benign or early stage cancer vs. malignant or late stage cancer; can be a known analyte level in an actual sample (*e.g*., a positive control); or can be a previously-determined analyte level for a particular individual (*e.g*., when monitoring an individual at two or more time points). The analyte is, for example, free NGAL.

"Test sample" includes a urine sample obtained from a subject being tested.

"Aggressive" or "invasive" includes the proclivity of a tumor for expanding beyond its boundaries into adjacent tissue (Darnell, J. (1990), Molecular Cell Biology, Third Ed., W.H. Freeman, NY). Invasive cancer can be contrasted with organ-confined cancer wherein the tumor is confined to a particular organ. The invasive property of a tumor is often accompanied by the elaboration of proteolytic enzymes, such as collagenases, that degrade matrix material and basement membrane material to enable the tumor to expand beyond the confines of the capsule, and beyond confines of the particular tissue in which that tumor is located.

"Metastasis" includes the condition of spread of cancer from the organ of origin to additional distal sites in the patient. The process of tumor metastasis is a multistage event involving local invasion and destruction of intercellular matrix, intravasation into blood vessels, lymphatics or other channels of transport, survival in the circulation, extravasation out of the vessels in the secondary site and growth in the new location (*see, e.g.,* Fidler, et al., Adv. Cancer Res. 28, 149-250 (1978), Liotta, et al., Cancer Treatment Res. 40, 223-238 (1988), Nicolson, Biochim. Biophy. Acta 948, 175-224 (1988) and Zetter, N. Eng. J. Med. 322, 605-612 (1990)). Increased malignant cell motility has been associated with enhanced metastatic potential in animal as well as human tumors (Hosaka, et al., Gann 69, 273-276 (1978) and Haemmerlin, et al., Int. J. Cancer 27, 603-610 (1981)).

A "primary tumor" includes a tumor appearing at a first site within the subject and can be distinguished from a "metastatic tumor" which appears in the body of the subject at a remote site from the primary tumor.

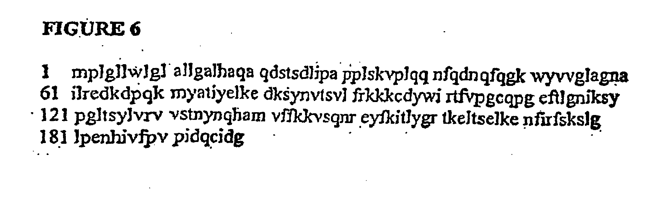

"NGAL" includes the NGAL protein of Genebank accession, Genpept, CAA58127 (Homo sapiens) (SEQ ID NO:1) (Fig. 6). NGAL is also referred to as neutrophil gelatinase-associated lipocalin and lipocalin-2. "NGAL" also encompasses species variants, homologues, allelic forms, mutant forms, and equivalents thereof.

The term 'higher level', as used in the context of the comparison(s) between the control sample(s) and the test sample(s) or control levels and test sample levels in the methods disclosed herein, includes a level that is statistically significant or significantly above levels found in the control sample, *e.g*., 1.3 fold higher or 1.5 fold higher. Preferably, the 'higher level' is at least 2 fold higher or greater.

"Statistically significant" or "significantly" includes statistical significance, and generally means a two standard deviation (2SD) above normal, or higher, concentration of the marker.

"LCIS" is lobular carcinoma *in situ.* LCIS is also called lobular neoplasia and is sometimes classified as a type of noninvasive breast cancer. It does not penetrate through the wall of the lobules. Although it does not itself usually become an invasive cancer, women with this condition have a higher risk of developing an invasive breast cancer in the same or opposite breast.

"DCIS" is ductal carcinoma *in situ.* Ductal carcinoma *in situ* is the most common type of noninvasive breast cancer. In DCIS, the malignant cells have not metastasized through the walls of the ducts into the fatty tissue of the breast. Comedocarcinoma is a type of DCIS that is more likely than other types of DCIS to come back in the same area after lumpectomy, and is more closely linked to eventual development of invasive ductal carcinoma than other forms of DCIS.

"Increased risk" of cancer includes a risk of cancer increased from that of the general population. A person said to have increased risk of cancer is in need of monitoring for the development of cancer and for the continued presence of cancer risk markers.

As described in further detail in this disclosure, cell lines derived from highly metastatic breast cancer, such as MDA-MB-231, express and secrete higher amounts of NGAL than cell lines derived from benign, organ defined breast cancers, such as T-47D and MCF-7. Furthermore, overexpression of NGAL, in a NGAL(-) breast cancer cell line (MCF-7), induces a mesenchymal-like phenotype, increases migration and invasion, and regulates Epithelial Mesenchymal Transition (EMT) marker transcription and expression, indicating a role for NGAL in metastatic disease.

The inventors have discovered that free urinary NGAL can be used as a biomarker to detect the presence and/or progression of cancer, *e.g*., cancer of epithelial origin including breast and ovarian cancer, and that non-invasive assays and tests can be used as an essential part of a cancer diagnosis and treatment. For those patients concerned about a cancer diagnosis, the non-invasive nature of the tests ameliorates somewhat the apprehension associated with the process. For doctors and care providers, testing urine in the manner described herein is rapid and reliable, and the methods of the invention utilize standard methodologies such as ELISA to obtain, *e.g*., a diagnosis of cancer or as part of a course of treatment (the therapeutic measures taken for a patient after diagnosis or after treatment for cancer) to determine whether a cancer is becoming more aggressive or metastasizing. Other cancers include peritoneal cancer, endometrial cancer, cervical cancer, colorectal cancer, uterine cancer, thyroid cancer, lung cancer, kidney cancer, and pancreatic cancer. For example, a determination of the likelihood for cancer recurrence, spread, or patient survival, can assist in determining whether a more conservative or more radical approach to therapy should be taken, or whether treatment modalities should be combined. For example, when cancer recurrence is likely, it can be advantageous to precede or follow surgical treatment with chemotherapy, radiation, immunotherapy, biological modifier therapy, gene therapy, vaccines, and the like, or adjust the span of time during which the patient is treated. The ease and speed with which the invention enables such insight is a decided advantage.

The methods disclosed herein may be used to determine whether or not an individual has a cancer of epithelial origin including breast and ovarian cancer, or is at increased risk of developing cancer. The methods involve measuring levels of free NGAL in a test sample obtained from an individual, and comparing the observed levels to control levels of free NGAL, *e.g*., those found in a control sample, or as control level is defined above. Levels of NGAL higher than control levels indicate that the individual is either at an increased risk of developing cancer, or has cancer. The levels of NGAL can be represented by arbitrary units, for example as units obtained from a densitometer, luminometer, or an ELISA plate reader.

For purposes of comparison, the test sample and control sample are of the same type, *i.e*., obtained from urine. However, the control sample can also be a standard sample that contains the same concentration of NGAL that is normally found in a urine sample obtained from a healthy individual.

The urine sample is preferably treated to prevent degradation of the NGAL protein. Methods for inhibiting or preventing degradation include, but are not limited to, treatment of the sample with protease inhibitors, freezing the sample, or placing the sample on ice. Preferably, prior to analysis, the samples are constantly kept under conditions as to prevent degradation of the NGAL protein.

A secondary diagnostic step can be performed. For example, if a level of NGAL is found to indicate either the risk or the presence of cancer, then an additional method of detecting the risk of cancer, or the presence of cancer can be performed to confirm. For example, to assess the risk of cancer, the individual can be tested for atypical ductal carcinoma (ADH) or lobular carcinoma *in situ* (LCIS) by means known to those in the art, such as atypia of the breast can be diagnosed by biopsy, random periareolar fine needle aspiration or ductal lavage. Genetic markers of risk can also be assessed, such as BRCA1 or BRCA2 or any other known genetic marker for cancer.

Any of a variety of additional diagnostic steps can be used to confirm the presence of cancer, such as ultrasound, mammography, PET scanning, MRI, thermal imaging, or any other imaging techniques, biopsy, ductal lavage, ductogram, clinical examination, or any other method known to those skilled in the art.

A patient with increased risk of cancer is preferably placed on a cancer detection regime, such as regular pap smears and mammographies with assessment of other risk markers. A patient may also be placed on preventative regimes, such as administration of angiogenesis inhibitors or selective hormone receptor modulators, preventive regimes are well known to those in the art. A patient with cancer is directed to cancer treatments well known to those in the art.

Additionally, disease progression can be assessed by following NGAL levels, such as free-NGAL levels, in an individual patient. For example, changes in an individual's condition can be monitored by comparing changes in NGAL expression levels in the individual over time, *e.g*., via standard methods for carrying out longitudinal studies in patients for the relevant condition. Progressive increases in NGAL levels are indicative of increased potential for having cancer, tumor invasion and metastasis, and/or a later stage of cancer, depending upon the reason for monitoring the individual over time. Alternatively, therapeutic efficacy of a treatment regimen can be monitored by monitoring the level of NGAL over time in an individual. For example, decreases in NGAL levels over time can be indicative of efficacy of the treatment regimen. Longitudinal monitoring can occur at two or more time points.

The methods outlined in the previous paragraph may also be advantageously used to longitudinally monitor patients who have been treated for cancer, *e.g*., for recurrence of the cancer. Since urinary NGAL, *e.g*., free NGAL, has been found in the present disclosure to be a useful cancer biomarker, an increased level of urinary NGAL in a patient post-treatment can be acted on accordingly, *e.g*., by further diagnosis, or further treatment, if appropriate. In post-treatment patients a control level of urinary NGAL would be established, *e.g*., using either one or several values of measured urinary NGAL, whichever is analytically appropriate. Changes in the post-treatment patients' condition can then be monitored by comparing changes in NGAL expression levels in the individual as needed. Depending on conditions appropriate for the patient, patient population or particular cancer, a progressive or gradual increase, or a rapid elevation, of urinary NGAL levels, can be indicative of a recurrence. Determining cancer recurrence can assist in determining whether a more conservative or more radical approach to therapy should be taken, or whether treatment modalities should be combined. When cancer recurrence is likely, it can be advantageous to precede or follow surgical treatment with chemotherapy, radiation, immunotherapy, biological modifier therapy, gene therapy, vaccines, and the like, or adjust the span of time during which the patient is treated.

Alternatively, urinary free NGAL levels in an individual being screened for recurrence of cancer can be compared against an empirically-determined control level. A test level from the individual greater than the control level indicates recurrence, which can be followed up with further therapy or monitoring, as described above.

### Measuring levels of free urinary NGAL

Free urinary NGAL levels may be measured preferably through the use of antibodies, or antibody equivalents, to detect free NGAL levels. Other methods, such as disclosed in WO 2004/088276, are also suitable; and NGAL levels may also be monitored by mass spectrometric analysis.

In one embodiment, levels of NGAL protein are measured by contacting the urine sample with an antibody-based binding moiety that preferentially binds to free NGAL, or to a fragment of free NGAL. Formation of the antibody-NGAL complex is then detected as a measure of free NGAL levels.

"Antibody-based binding moiety" or "antibody" includes immunoglobulin molecules and immunologically active determinants of immunoglobulin molecules, *e.g*., molecules that contain an antigen binding site which preferentially binds (immunoreacts with) to NGAL, or to the additional biomarkers of the invention. "Antibody-based binding moiety includes whole antibodies, *e.g*., of any isotype (IgG, IgA, IgM, IgE, etc), and fragments thereof which are also specifically reactive with NGAL protein, more preferably those which preferentially bind to uncomplexed (free) NGAL. Antibodies can be fragmented using conventional techniques. Thus, the term includes segments of proteolytically-cleaved or recombinantly-prepared portions of an antibody molecule that are capable of selectively reacting with a certain protein. Non-limiting examples of such proteolytic and/or recombinant fragments include Fab, F(ab')2, Fab' , Fv, dAbs and single chain antibodies (scFv) containing a VL and VH domain joined by a peptide linker. The scFvs may be covalently or non-covalently linked to form antibodies having two or more binding sites. Thus, "antibody-based binding moiety" includes polyclonal, monoclonal, or other purified preparations of antibodies and recombinant antibodies. The term "antibody-based binding moiety" is further intended to include humanized antibodies, bispecific antibodies, and chimeric molecules having at least one antigen binding determinant derived from an antibody molecule. In a preferred embodiment, the antibody-based binding moiety is detectably labeled.

"Labeled antibody" includes antibodies that are labeled by a detectable means and include antibodies that are enzymatically, radioactively, fluorescently, and chemiluminescently labeled. Antibodies can also be labeled with a detectable tag, such as c-Myc, HA, VSV-G, HSV, FLAG, V5, or HIS.

In the diagnostic and prognostic methods of the invention that use antibody-based binding moieties for the detection of NGAL, the level of free NGAL protein present in the urine samples correlate to the intensity of the signal emitted from the detectably labeled antibody.

In one preferred embodiment, the antibody-based binding moiety is detectably labeled by linking the antibody to an enzyme. The enzyme, in turn, when exposed to its substrate, will react with the substrate in such a manner as to produce a chemical moiety which can be detected, for example, by spectrophotometric, fluorometric or by visual means. Enzymes which can be used to detectably label the antibodies of the present invention include malate dehydrogenase, staphylococcal nuclease, delta-V-steroid isomerase, yeast alcohol dehydrogenase, alpha-glycerophosphate dehydrogenase, triose phosphate isomerase, horseradish peroxidase, alkaline phosphatase, asparaginase, glucose oxidase, beta-galactosidase, ribonuclease, urease, catalase, glucose-VI-phosphate dehydrogenase, glucoamylase and acetylcholinesterase. Chemiluminescence is another method that can be used to detect an antibody-based binding moiety.

Detection may also be accomplished using any of a variety of other immunoassays. For example, by radioactively labeling an antibody, it is possible to detect the antibody through the use of radioimmunoassays. The radioactive isotope can be detected by such means as the use of a gamma counter or a scintillation counter or by autoradiography. Isotopes which are particularly useful for the purpose of the present invention are ³H, ¹³¹I, ³⁵S, ¹⁴C, and preferably ¹²⁵I.

It is also possible to label an antibody with a fluorescent compound. When the fluorescently labeled antibody is exposed to light of the proper wave length, its presence can then be detected due to fluorescence. Among the most commonly used fluorescent labeling compounds are CYE dyes, fluorescein isothiocyanate, rhodamine, phycoerytherin, phycocyanin, allophycocyanin, o-phthaldehyde and fluorescamine.

An antibody can also be detectably labeled using fluorescence emitting metals such as ¹⁵²Eu, or others of the lanthanide series. These metals can be attached to the antibody using such metal chelating groups as diethylenetriaminepentaacetic acid (DTPA) or ethylenediaminetetraacetic acid (EDTA).

An antibody also can be detectably labeled by coupling it to a chemiluminescent compound. The presence of the chemiluminescent-antibody is then determined by detecting the presence of luminescence that arises during the course of a chemical reaction. Examples of particularly useful chemiluminescent labeling compounds are luminol, luciferin, isoluminol, theromatic acridinium ester, imidazole, acridinium salt and oxalate ester.

As discussed above, free NGAL levels can be detected by immunoassays, such as enzyme linked immunoabsorbant assay (ELISA), radioimmunoassay (RIA), Immunoradiometric assay (IRMA), Western blotting, or immunohistochemistry, each of which are described in more detail below. Antibody arrays or protein chips can also be employed, see for example U.S. Patent Publication Nos. 20030013208A1; 20020155493A1; and 20030017515, and U.S. Patent Nos: 6,329,209 and 6,365,418.

### Immunoassays

"Radioimmunoassay" is a technique for detecting and measuring the concentration of an antigen using a labeled (*e.g*., radioactively labeled) form of the antigen. Examples of radioactive labels for antigens include ³H, ¹⁴C, and ¹²⁵I. The concentration of free NGAL antigen in a biological sample is measured by having the antigen in the biological sample compete with the labeled (*e.g*., radioactively) antigen for binding to an antibody to the antigen. To ensure competitive binding between the labeled antigen and the unlabeled antigen, the labeled antigen is present in a concentration sufficient to saturate the binding sites of the antibody. The higher the concentration of antigen in the sample, the lower the concentration of labeled antigen that will bind to the antibody.

In a radioimmunoassay, to determine the concentration of labeled antigen bound to antibody, the antigen-antibody complex must be separated from the free antigen. One method for separating the antigen-antibody complex from the free antigen is by precipitating the antigen-antibody complex with an anti-isotype antiserum. Another method for separating the antigen-antibody complex from the free antigen is by precipitating the antigen-antibody complex with formalin-killed S. aureus. Yet another method for separating the antigen-antibody complex from the free antigen is by performing a "solid-phase radioimmunoassay" where the antibody is linked (*e.g*., covalently) to Sepharose beads, polystyrene wells, polyvinylchloride wells, or microtiter wells. By comparing the concentration of labeled antigen bound to antibody to a standard curve based on samples having a known concentration of antigen, the concentration of antigen in the biological sample can be determined.

A "Immunoradiometric assay" (IRMA) is an immunoassay in which the antibody reagent is radioactively labeled. An IRMA requires the production of a multivalent antigen conjugate, by techniques such as conjugation to a protein *e.g*., rabbit serum albumin (RSA). The multivalent antigen conjugate must have at least 2 antigen residues per molecule and the antigen residues must be of sufficient distance apart to allow binding by at least two antibodies to the antigen. For example, in an IRMA the multivalent antigen conjugate can be attached to a solid surface such as a plastic sphere. Unlabeled "sample" antigen and antibody to antigen which is radioactively labeled are added to a test tube containing the multivalent antigen conjugate coated sphere. The antigen in the sample competes with the multivalent antigen conjugate for antigen antibody binding sites. After an appropriate incubation period, the unbound reactants are removed by washing and the amount of radioactivity on the solid phase is determined. The amount of bound radioactive antibody is inversely proportional to the concentration of antigen in the sample.

The most common enzyme immunoassay is the "Enzyme-Linked Immunosorbent Assay (ELISA)." ELISA is a technique for detecting and measuring the concentration of an antigen using a labeled (*e.g*., enzyme linked) form of the antibody. There are different forms of ELISA, which are well known to those skilled in the art. The standard techniques known in the art for ELISA are described in "Methods in Immunodiagnosis", 2nd Edition, Rose and Bigazzi, eds. John Wiley & Sons, 1980; Campbell et al., "Methods and Immunology", W. A. Benjamin, Inc., 1964; and Oellerich, M. 1984, J. Clin. Chem. Clin. Biochem. 22:895-904.

In a "sandwich ELISA", an antibody (*e.g*., anti-NGAL or anti-free NGAL) is linked to a solid phase (*i.e*., a microtiter plate) and exposed to a biological sample containing antigen (*e.g*., NGAL). The solid phase is then washed to remove unbound antigen. A labeled antibody (*e.g*., enzyme linked) is then bound to the bound-antigen (if present) forming an antibody-antigen-antibody sandwich. Examples of enzymes that can be linked to the antibody are alkaline phosphatase, horseradish peroxidase, luciferase, urease, and β-galactosidase. The enzyme linked antibody reacts with a substrate to generate a colored reaction product that can be measured.

In a "competitive ELISA", antibody (*e.g*., anti-NGAL or anti-free NGAL) is incubated with a sample containing antigen (*i.e*., NGAL). The antigen-antibody mixture is then contacted with a solid phase (*e.g*., a microtiter plate) that is coated with antigen (*i.e.*, NGAL). The more antigen present in the sample, the less free antibody that will be available to bind to the solid phase. A labeled (*e.g*., enzyme linked) secondary antibody is then added to the solid phase to determine the amount of primary antibody bound to the solid phase.

In a "immunohistochemistry assay" a section of tissue is tested for specific proteins by exposing the tissue to antibodies that are specific for the protein that is being assayed. The antibodies are then visualized by any of a number of methods to determine the presence and amount of the protein present. Examples of methods used to visualize antibodies are, for example, through enzymes linked to the antibodies (*e.g*., luciferase, alkaline phosphatase, horseradish peroxidase, or β-galactosidase), or chemical methods (*e.g*., DAB/Substrate chromagen).

Other techniques may be used to detect the biomarkers of the invention, according to a practitioner's preference, and based upon the present disclosure. One such technique is Western blotting (Towbin et at., Proc. Nat. Acad Sci. 76:4350 (1979)), wherein a suitably treated sample is run on an SDS-PAGE gel before being transferred to a solid support, such as a nitrocellulose filter. Detectably labeled antibodies that preferentially bind to NGAL (*e.g.*, anti-NGAL or anti-free NGAL) can then be used to assess NGAL levels, where the intensity of the signal from the detectable label corresponds to the amount of NGAL present. Levels can be quantitated, for example by densitometry.

### Mass Spectrometry

In addition, NGAL (*e.g*., free NGAL) may be detected using Mass Spectrometry such as MALDI/TOF (time-of-flight), SELDI/TOF, liquid chromatography-mass spectrometry (LC-MS), gas chromatography-mass spectrometry (GC-MS), high performance liquid chromatography-mass spectrometry (HPLC-MS), capillary electrophoresis-mass spectrometry, nuclear magnetic resonance spectrometry, or tandem mass spectrometry (*e.g*., MS/MS, MS/MS/MS, ESI-MS/MS, etc.). *see, e.g.,* U.S. Publication Nos. 20030199001, 20030134304, and 20030077616.

Mass spectrometry methods are well known in the art and have been used to quantify and/or identify proteins (see, *e.g.,* Li et al. (2000) Tibtech 18:151-160; Rowley et al. (2000) Methods 20: 383-397; and Kuster and Mann (1998) Curr. Opin. Structural Biol. 8: 393-400). Further, mass spectrometric techniques have been developed that permit at least partial de novo sequencing of isolated proteins. Chait et al., Science 262:89-92 (1993); Keough et al., Proc. Natl. Acad. Sci. USA. 96:7131-6 (1999); reviewed in Bergman, EXS 88:133-44 (2000).

In certain embodiments, a gas phase ion spectrophotometer is used. In other embodiments, laser-desorption/ionization mass spectrometry is used to analyze the sample. Modem laser desorption/ionization mass spectrometry ("LDI-MS") can be practiced in two main variations: matrix assisted laser desorption/ionization ("MALDI") mass spectrometry and surface-enhanced laser desorption/ionization ("SELDI"). In MALDI, the analyte is mixed with a solution containing a matrix, and a drop of the liquid is placed on the surface of a substrate. The matrix solution then co-crystallizes with the biological molecules. The substrate is inserted into the mass spectrometer. Laser energy is directed to the substrate surface where it desorbs and ionizes the biological molecules without significantly fragmenting them. However, MALDI has limitations as an analytical tool. It does not provide means for fractionating the sample, and the matrix material can interfere with detection, especially for low molecular weight analytes. *See, e.g.,* U.S. Pat. Nos. 5,118,937 and 5,045,694.

In SELDI, the substrate surface is modified so that it is an active participant in the desorption process. In one variant, the surface is derivatized with adsorbent and/or capture reagents that selectively bind the protein of interest. In another variant, the surface is derivatized with energy absorbing molecules that are not desorbed when struck with the laser. In another variant, the surface is derivatized with molecules that bind the protein of interest and that contain a photolytic bond that is broken upon application of the laser. In each of these methods, the derivatizing agent generally is localized to a specific location on the substrate surface where the sample is applied. *See, e.g.,* U.S. Pat. No. 5,719,060 and WO 98/59361. The two methods can be combined by, for example, using a SELDI affinity surface to capture an analyte and adding matrix-containing liquid to the captured analvte to provide the energy absorbing material.

For additional information regarding mass spectrometers, *see, e.g.,* Principles of Instrumental Analysis, 3rd edition., Skoog, Saunders College Publishing, Philadelphia, 1985; and Kirk-Othmer Encyclopedia of Chemical Technology, 4th ed. Vol. 15 (John Wiley & Sons, New York 1995), pp. 1071-1094.

Detection of the presence of a marker or other substances will typically involve detection of signal intensity. This, in turn, can reflect the quantity and character of a polypeptide bound to the substrate. For example, in certain embodiments, the signal strength of peak values from spectra of a first sample and a second sample can be compared (*e.g*., visually, by computer analysis etc.), to determine the relative amounts of particular biomolecules. Software programs such as the Biomarker Wizard program (Ciphergen Biosystems, Inc., Fremont, Calif.) can be used to aid in analyzing mass spectra. The mass spectrometers and their techniques are well known to those of skill in the art.

Any person skilled in the art understands, any of the components of a mass spectrometer (*e.g*., desorption source, mass analyzer, detect, etc.) and varied sample preparations can be combined with other suitable components or preparations described herein, or to those known in the art. For example, in some embodiments a control sample may contain heavy atoms (*e.g*. ¹³C) thereby permitting the test sample to be mixed with the known control sample in the same mass spectrometry run.

In one preferred embodiment, a laser desorption time-of-flight (TOF) mass spectrometer is used. In laser desorption mass spectrometry, a substrate with a bound marker is introduced into an inlet system. The marker is desorbed and ionized into the gas phase by laser from the ionization source. The ions generated are collected by an ion optic assembly, and then in a time-of-flight mass analyzer, ions are accelerated through a short high voltage field and let drift into a high vacuum chamber. At the far end of the high vacuum chamber, the accelerated ions strike a sensitive detector surface at a different time. Since the time-of-flight is a function of the mass of the ions, the elapsed time between ion formation and ion detector impact can be used to identify the presence or absence of molecules of specific mass to charge ratio.

In some embodiments the relative amounts of one or more biomolecules present in a first or second sample is determined, in part, by executing an algorithm with a programmable digital computer. The algorithm identifies at least one peak value in the first mass spectrum and the second mass spectrum. The algorithm then compares the signal strength of the peak value of the first mass spectrum to the signal strength of the peak value of the second mass spectrum. The relative signal strengths are an indication of the amount of the biomolecule (*e.g*., NGAL or free NGAL) that is present in the first and second samples. A standard containing a known amount of a biomolecule can be analyzed as the second sample to better quantify the amount of the biomolecule present in the first sample. In certain embodiments, the identity of the biomolecules in the first and second sample can also be determined. In one preferred embodiment, biomarker levels are measured by MALDI-TOF mass spectrometry.

### Antibodies

The free NGAL-binding antibodies for use in the invention can be obtained from a commercial source (for example, the anti-free NGAL antibody available in NGAL ELISA kit #DLCN20 from R&D Systems, Minneapolis, MN USA.) Alternatively, antibodies can be raised against free NGAL, or a portion of the biomarker polypeptide, *e.g*., an amino acid portion that is normally masked in the MMP-9/NGAL complex, in order to generate an antibody that only interacts with free NGAL and not MMP-9/NGAL. Such antibody-binding moieties may be raised against an epitope inside the MMP-9/NGAL binding region by those of ordinary skill in the art, and need not be disclosed in further detail here.

Antibodies for use in the present invention can be produced using standard methods to produce antibodies, for example, by monoclonal antibody production (Campbell, A.M., Monoclonal Antibodies Technology: Laboratory Techniques in Biochemistry and Molecular Biology, Elsevier Science Publishers, Amsterdam, the Netherlands (1984); St. Groth et al., J. Immunology, (1990) 35: 1-21; and Kozbor et al., Immunology Today (1983) 4:72). Antibodies can also be readily obtained by using antigenic portions of the protein to screen an antibody library, such as a phage display library by methods well known in the art. For example, U.S. Patent No. 5,702,892 and WO 01/18058 disclose bacteriophage display libraries and selection methods for producing antibody binding domain fragments.

### NGAL Detection Kits

Commercial kits for the detection and prognostic evaluation of ovarian cancer and for the detection and prognostic evaluation of breast cancer are provided according to this disclosure. The kits may be in any configuration well known to those of ordinary skill in the art and are useful for performing one or more of the methods described herein for the detection of NGAL. The kits are convenient in that they supply many, if not all, of the essential reagents for conducting an assay for the detection of NGAL in a urine sample. In addition, the assay is preferably performed simultaneously with a standard or multiple standards that are included in the kit, such as a predetermined amount of NGAL protein, so that the results of the test can be quantitated or validated.

The kits include the means for detecting NGAL levels *i.e.,* NGAL binding antibodies or antibody fragments which selectively bind to NGAL protein. The diagnostic assay kit is preferentially formulated in a standard two-antibody binding format, in which one NGAL specific antibody captures NGAL in a patient sample and another NGAL specific antibody is used to detect captured NGAL. For example, the capture antibody is immobilized on a solid phase, *e.g*., an assay plate, an assay well, a nitrocellulose membrane, a bead, a dipstick, or a component of an elution column. The second antibody, *i.e*., the detection antibody, is typically tagged with a detectable label such as a calorimetric agent or radioisotope.

In one preferred embodiment, the kit comprises a means for detecting levels of free NGAL in a sample of urine. In a specific embodiment, the kit comprises a "dipstick" with anti-NGAL antibodies or fragments, immobilized thereon, which preferentially bind NGAL protein. Preferentially bound NGAL protein can then be detected using, for example, a second antibody that is detectably labeled with a colorimetric agent or radioisotope, whereupon the amount of free NGAL may be quantitatively determined as those of ordinary skill in the art will understand.

In other embodiments, the assay kits may employ (but are not limited to) the following techniques: competitive and non-competitive assays, radioimmunoassay (RIA) , bioluminescence and chemiluminescence assays, fluorometric assays, sandwich assays, immunoradiometric assays, dot blots, enzyme linked assays including ELISA, microtiter plates, and immunocytochemistry. For each kit the range, sensitivity, precision, reliability, specificity and reproducibility of the assay are established by means well known to those skilled in the art.

The present invention is further illustrated by the following Examples. These examples are provided to aid in the understanding of the invention and are not construed as a limitation thereof.

### EXAMPLE 1

### NGAL as a urinary biomarker for ovarian and breast cancer

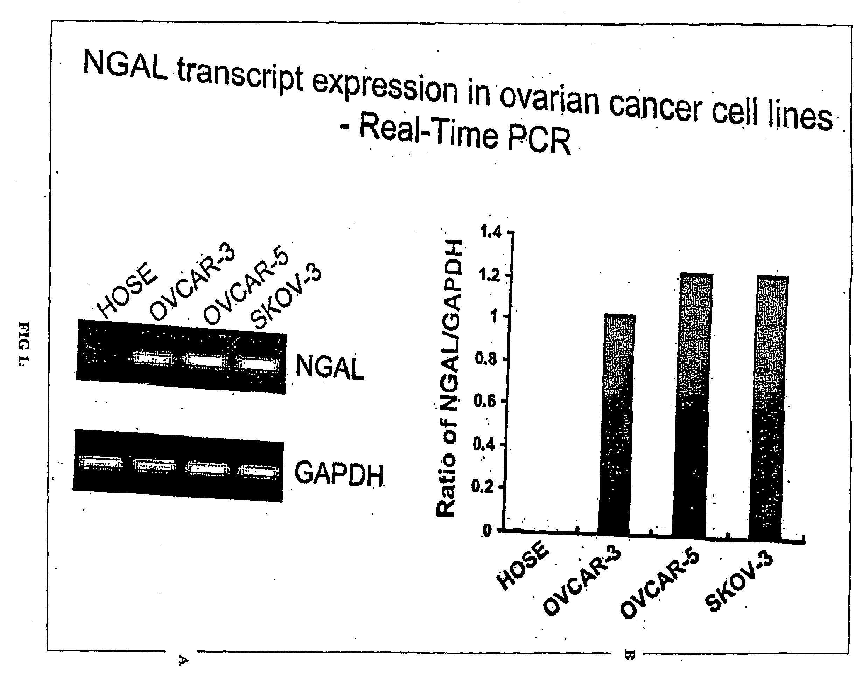

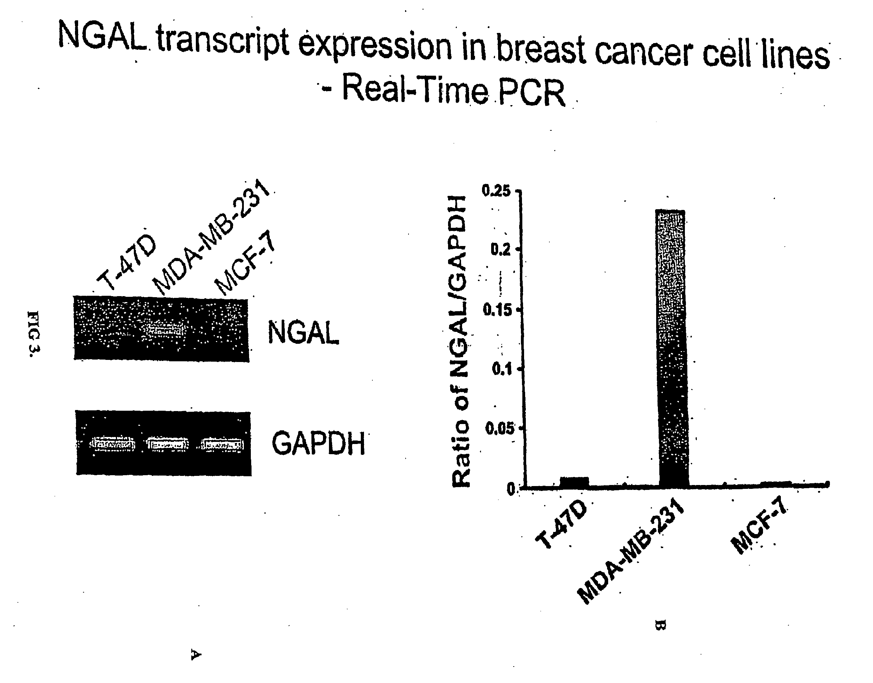

This example shows that free NGAL can serve as a biomarker for cancer, *e.g*., of epithelial origin such as breast and/or ovarian cancer. Moreover, it demonstrates that free NGAL is useful for diagnosing, prognosing and staging of cancers. Figures 1a and 1b show the NGAL transcript expression levels in various ovarian cancer cell lines. Figures 3A and 3B show the NGAL transcript expression levels in various breast cancer cell lines. Expression levels were determined using real-time PCR. These results confirm that NGAL is overexpressed in cancer cell lines as compared to non-cancerous (HOSE) cell lines.

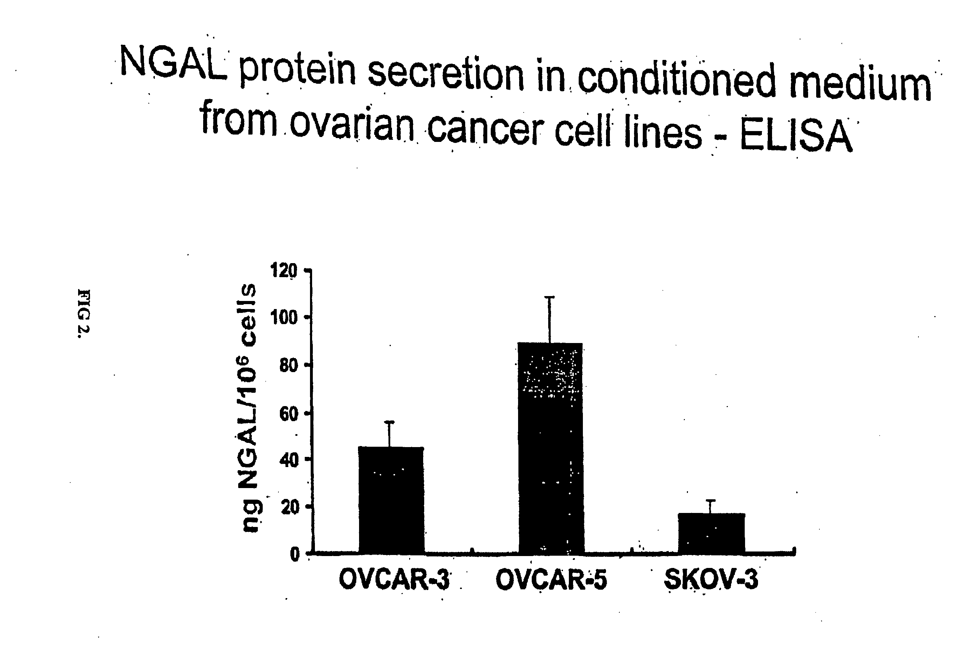

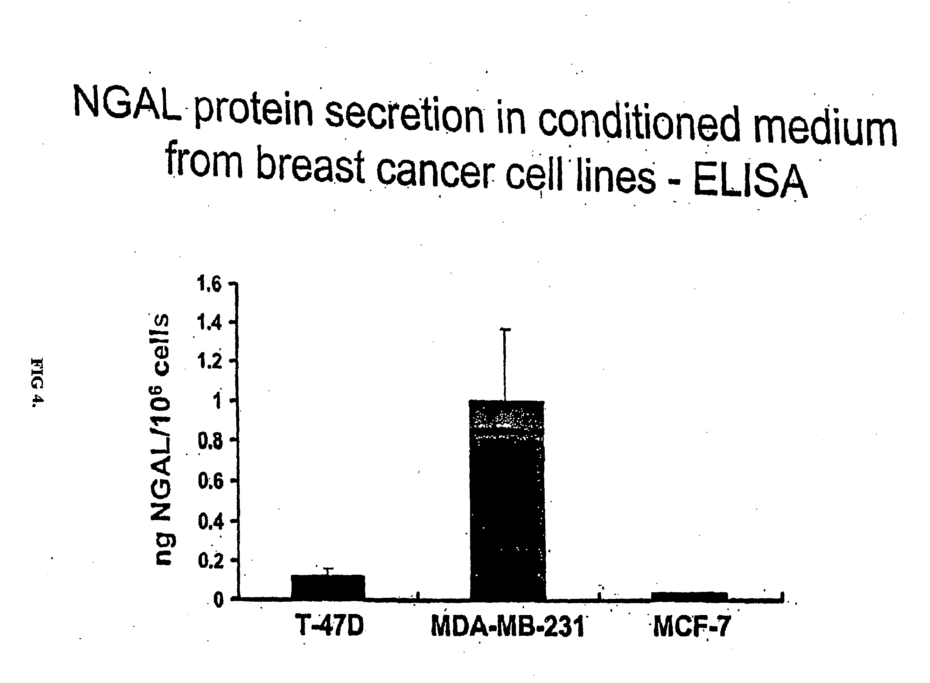

Figure 2 shows a graph illustrating the amount of NGAL protein secretion in conditioned medium from ovarian cancer cell lines as determined by Borregaard ELISA as described in Kjeldsen L, Koch C, Arnljots K, Borregaard N., Characterization of two ELISAs for NGAL, a newly described lipocalin in human neutrophils. J Immunol Methods. 1996 Nov 13;198(2):155-64, using anti-NGAL antibody provided by Dr. Borregaard. OVCAR-5 shows the highest level of secretion while SKOV-3 had significantly less secretion than OVCAR-5. Figure 4 shows a graph illustrating the amount of NGAL protein secretion in conditioned medium from breast cancer cell lines using Borregaard ELISA. MDA-MB-231, a cell line derived from a highly metastatic cancer, shows the highest level of NGAL secretion, while there is significantly less secretion in cell lines derived from organ confined cancers (MCF-7 and T-47D).

However, the inventors show in the data below that, free urinary NGAL protein serves a biomarker for breast and/or ovarian cancer. In particular, there is an increase in levels of free NGAL in urine samples from patients that have malignant cancer, as compared to control urine samples from patients that do not have cancer.

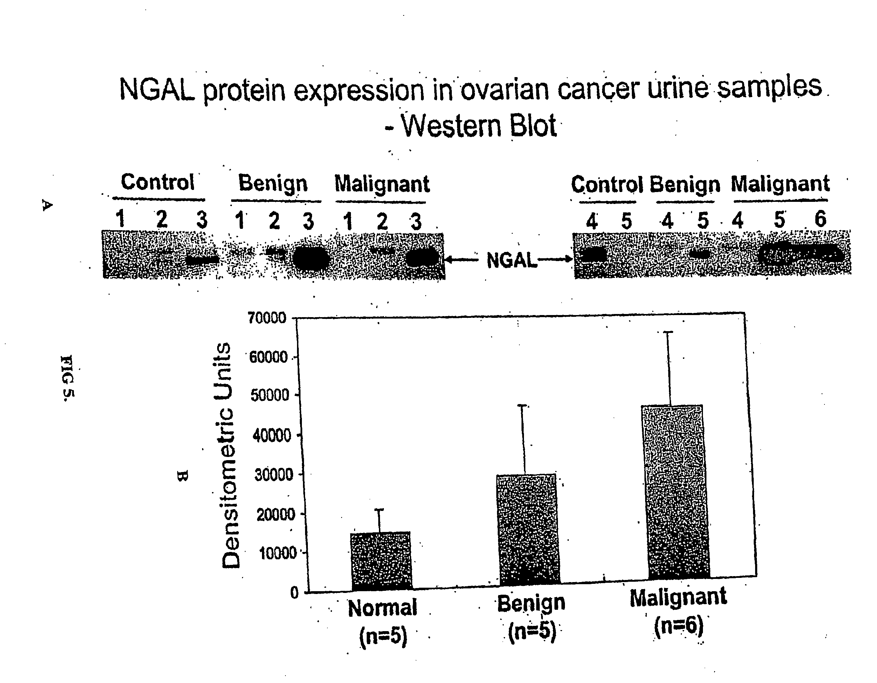

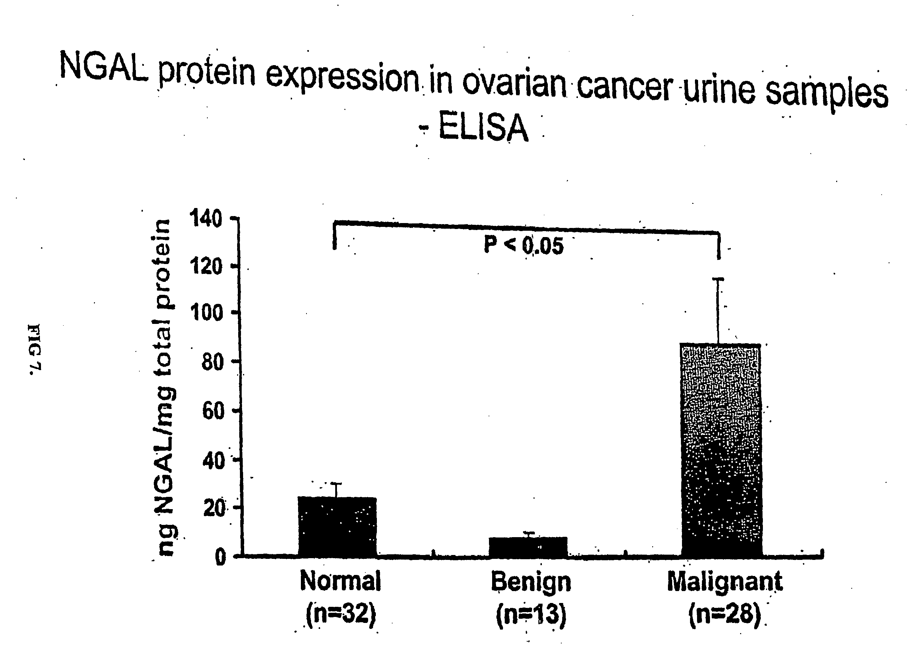

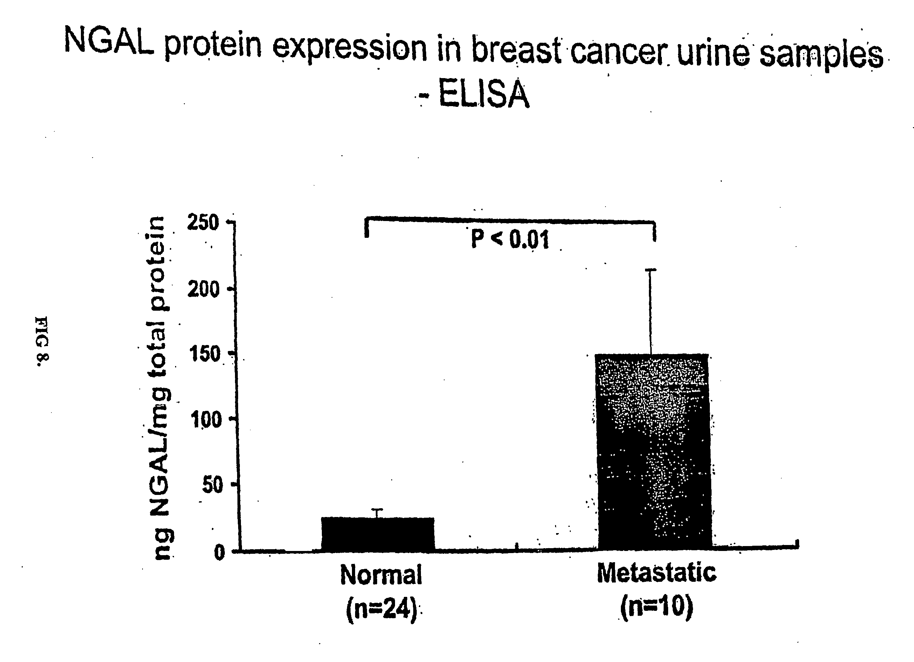

The levels of NGAL protein in urine samples from patients with either no disease, benign ovarian disease, or malignant ovarian cancer was determined using Western blot analysis (Figures 5A and 5B). The SDS-PAGE run under reducing conditions, measuring both free and complexed NGAL. Figure 5A shows a Western blot analysis of urine samples using anti-human NGAL from R&D Systems (Minneapolis, MN USA). Figure 5B shows the quantitative analysis of the results from at least 5 samples of urine from patients with either no disease, benign ovarian disease, or malignant ovarian cancer. Patients with malignant ovarian cancer showed higher amounts of NGAL in urine as compared to samples from individuals without disease. This was confirmed by a Borregaard ELISA assay (Figure 7). Similar analysis of urine samples from breast cancer patients also showed higher levels of NGAL in urine from patients that have malignant cancer as compared to patients without disease (Figure 8).

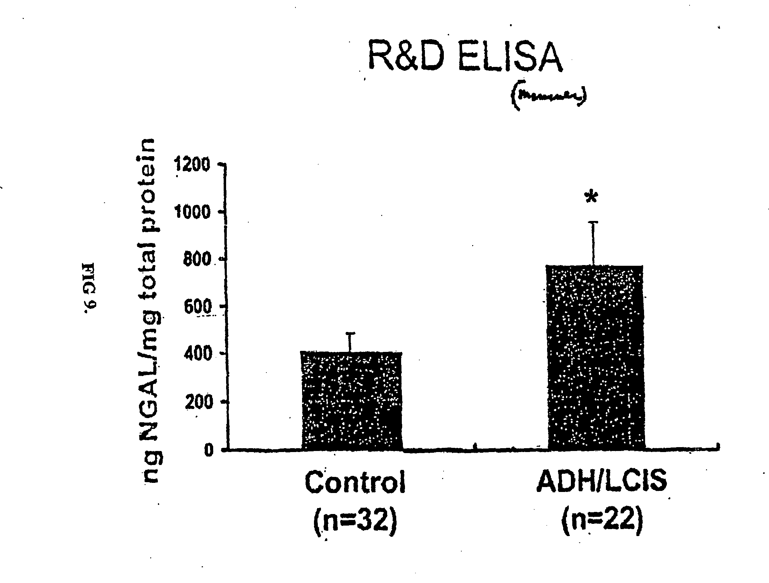

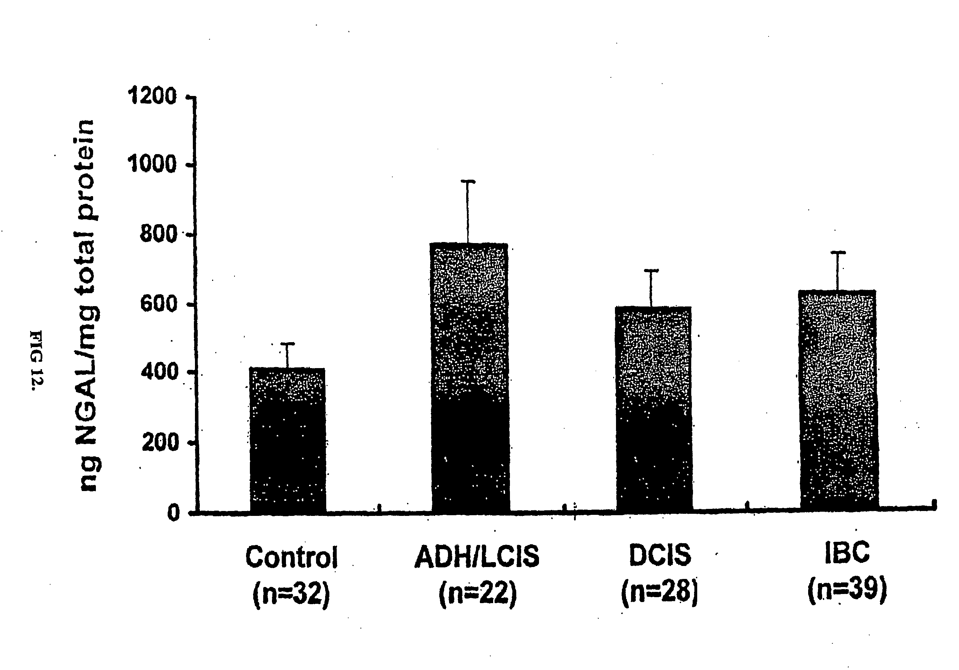

It was further discovered that individuals with atypical ductal hyperplasia (ADH), a major risk factor for future breast cancer development, have higher levels of free NGAL in urine than levels seen in healthy individuals. An ELISA assay that uses an anti-NGAL antibody, which recognizes only free NGAL (from NGAL ELISA kit #DLCN20 from R&D Systems, Minneapolis, MN USA), was used to assess the levels of free NGAL, the results are shown in Figure 9. Using R&D ELISA, increased levels of free NGAL were also detected in individuals with ductal carcinoma in situ (DCIS), the most common type of noninvasive breast cancer, and with invasive breast cancer (IBC), as compared to levels seen in healthy individuals. (Figure 12).

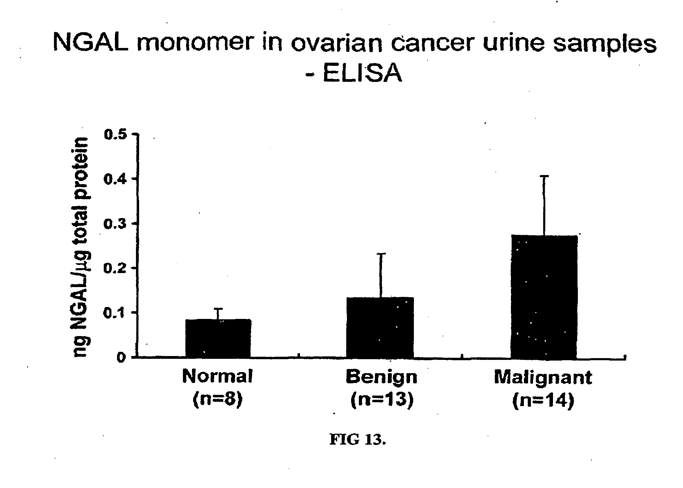

Free NGAL is present in urine in at least monomeric, dimeric and trimeric forms. Detection of free monomeric urinary NGAL is possible in accordance with the present disclosure. In this instance, the studies were carried out with NGAL ELISA kit #DLCN20 from R&D Systems, Minneapolis, MN USA. The presence of monomeric NGAL appears to be particularly indicative of benign and malignant ovarian cancers, as shown in Figure 13. Figure 13 is a graph showing the amount of free NGAL monomer present in ovarian cancer urine samples, from patients having benign and malignant tumors, in comparison with normal samples. The malignant tumor samples evidence a much higher free NGAL level than the benign sample population.

Thus, measuring the level of free NGAL in urine provides a quick, easy, and safe screen that can be used to determine whether or not an individual either has a cancer, *e.g*., of epithelial origin such as breast and/or ovarian cancer, or is at an increased risk for developing a cancer. A higher level of free NGAL in urine as compared to an age and sex matched control sample indicates that the individual either has a cancer or is at increased risk for developing a breast and/or ovarian cancer. A positive test indicates that the individual should be tested further for cancer and monitored accordingly.

### EXAMPLE 2

### NGAL involvement in Epithelial Mesenchymal Transition (EMT)

Epithelial Mesenchymal Transition (EMT) is an important process by which epithelial cells acquire mesenchymal, fibroblast-like properties and show reduced intercellular adhesion and increased motility. It is believed that EMT-like events occur during tumor progression and malignant transformation, endowing cancer cells with invasive and metastatic properties (Laruel et al. Oncogene (2005) 24, 7443-7454).

To assess the potential role of NGAL in the development of aggressive cancer, we prepared stable MCF-7 cell lines that overexpress NGAL, N1 and N2. MCF-7 is a breast cancer cell line that expresses little NGAL. We found that the N1 and N2 cell clones (MCF-7 cells that overexpress NGAL) exhibit a mesenchymal-like phenotype, these cells lose cell-contacts and scatter more evenly across the cell surface (data not shown).

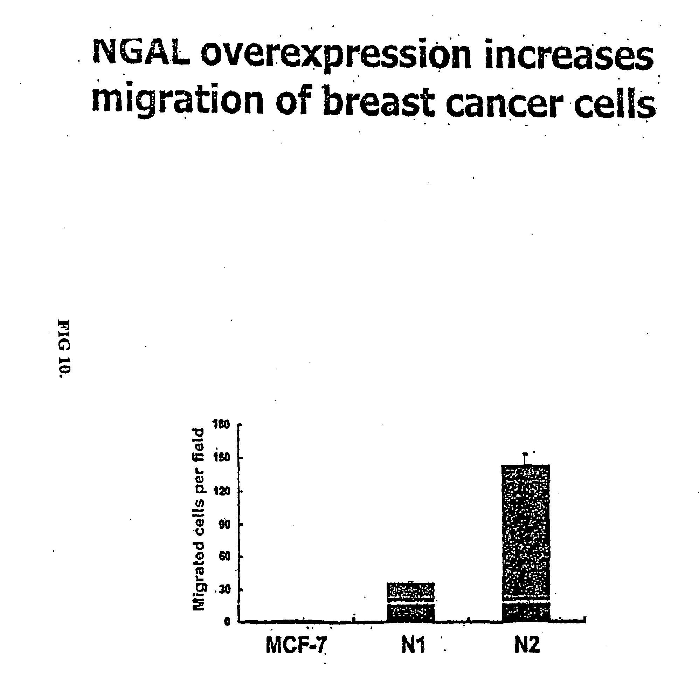

The migration of NGAL overexpressing cells was evaluated using BD Falcon HTS FluoroBlok 24-Multiwell Insert System (BD Bioscience, Bedford, MA) according to manufacture's manual. MCF-7 control and NGAL-overexpressing cells (N1/N2) were grown to 70% confluence and were then trypsinized and resuspended in serum-free medium. 5×10⁴ cells were seeded into the top chambers. Full media (750µl) were used as chemoattractant and were added into the lower chambers. After incubation in 37°C, 5% CO₂ incubator for 24 hours, medium was removed from upper chambers. The insert plates were then transferred to a new 24-well plate containing 0.5ml/well of 10µM CellTracker CMFDA (Molecular Probes) and were incubated for 1 hour at 37°C, 5% CO₂. The BD FluoroBlok membrane only allows the labeled cells that migrated through the membrane to be visualized under fluorescence microscope. The results of this assay clearly show that overexpression of NGAL increases motility. Cells overexpressing NGAL migrated more than the parental MCF-7 cells (Figure 10).

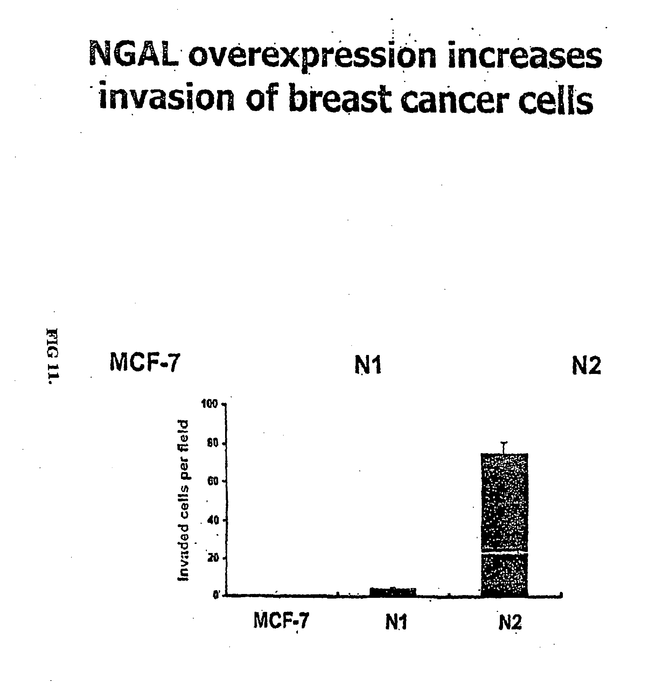

In addition, cell invasion was stimulated by overexpression of NGAL. The invasiveness of NGAL overexpressing cells was evaluated using a 24-well BD BioCoat Tumor Invasion System (BD Bioscience, Bedford, MA) according to manufacture's manual. In this system, the FluoroBlok membranes are covered with a layer of Matrigel™. MCF-7 control and NGAL-overexpressing cells (N1/N2) were grown to 70% confluence and were then trypsinized and resuspended in serum-free medium. 5×10⁴ cells were seeded into the top chambers. Full media (750µl) were used as chemoattractant and were added into the lower chambers. After incubation in 37°C, 5% CO₂ incubator for 24 hours, medium was removed from upper chambers. The insert plates were then transferred to a new 24-well plate containing 0.5ml/well of 10µM CellTracker CMFDA (Molecular Probes) and were incubated for 1 hour at 37°C, 5% CO₂. The BD FluoroBlok membrane only allows the labeled cells that invaded through BD Matrigel™ membrane to be visualized under fluorescence microscope. The results of this assay show that overexpression of NGAL increases invasiveness (Figure 11).

We also assessed the ability of NGAL to regulate molecules known to be involved in Epithelial Mesenchymal Transition (EMT). Using RT-PCR and the cell lines that overexpress NGAL, we found that overexpression of NGAL increases the expression of the mesenchymal markers vimentin and fibronectin (data not shown). Overexpression of NGAL reduces ERα and E-cadherin expression while it increases expression of Slug, a transcription factor that regulates EMT.

Thus, NGAL is involved in the development of cell phenotypes consistent with what is found in aggressive cancers.

### EQUIVALENTS

Those skilled in the art will recognize, or be able to ascertain using no more than routine experimentation, numerous equivalents to the specific procedures described herein. Such equivalents are considered to be within the scope of the invention and are covered by the following claims.

### REFERENCES .

Santin et al., Int. J. Cancer 2004, 112: 14-25. Gene expression profiles in primary ovarian serous papillary tumors and normal ovarian epithelium: Identification of candidate molecular markers for ovarian cancer diagnosis and therapy.

Mishara et al, J. Am. Soc. Nephrol. 14:2534-2543 Identification of neutrophil gelatinase associated lipocalin as a novel early urinary biomarker for ischemic renal injury.

O'Brien et al, Experimental. Dermatology 2002, 11: 584-591 Neutrophil gelatinase-associated lipocalin is a marker for dysregulated keratinocyte differentiation in human skin.

## Claims

1. A method for determining if an individual is at risk of developing, or has developed, cancer, comprising the steps of:
a) determining a level of free NGAL in a test urine sample obtained from an individual;
b) comparing the level of free NGAL in the test urine sample with a control level of free NGAL;
wherein a level of free NGAL in the test sample higher than a control level of free NGAL indicates that the individual is either at increased risk for developing cancer, or has cancer;
wherein the cancer is a cancer of epithelial origin.

2. The method of claim 1, wherein the cancer of epithelial origin is selected from the group consisting of breast cancer, basal cell carcinoma, adenocarcinoma, gastrointestinal cancer, lip cancer, mouth cancer, esophageal cancer, small bowel cancer, stomach cancer, colon cancer, liver cancer, bladder cancer, pancreas cancer, ovary cancer, cervical cancer, lung cancer, skin cancer, kidney cancer, prostate cancer, and renal cell carcinoma.

3. The method of claim 1, wherein the cancer is breast and/or ovarian cancer.

4. The method of claim 1, wherein the level of free NGAL is measured by a method comprising the steps of:
a) contacting the test sample, or preparation thereof, with an antibody-based binding moiety which preferentially binds to free NGAL to form an antibody- NGAL complex; and
b) detecting the presence of the antibody-NGAL complex, thereby measuring the level of free NGAL present.

5. The method of claim 1, wherein the antibody-based binding moiety is labeled with a detectable label.

6. The method of claim 1, wherein the label is selected from the group consisting of a radioactive label, a hapten label, a fluorescent label, and an enzymatic label.

7. The method of claim 1, wherein the antibody-based binding moiety is an antibody.

8. The method of claim 1, wherein the antibody is a monoclonal antibody.

9. The method of claim 1, further comprising the step of reviewing the results and if the urine has a higher level of free NGAL than the control level of free NGAL, directing the individual to be further tested for cancer.

10. The method of claim 1, wherein the level of free NGAL is measured by mass spectrophotometric methods.

11. The method of claim 1, wherein the individual is a post-cancer treatment patient, and when a level of free NGAL in the test sample is higher than a control level of free NGAL, the post-cancer treatment patient is referred for treatment of recurrence of the cancer.

12. A method for staging a cancer of epithelial origin, comprising the steps of:
a) determining a level of free NGAL in a test urine sample obtained from an individual;
b) comparing the level of free NGAL in the test urine sample with a control level of free NGAL;
wherein a level of free NGAL in the test sample higher than a control level of free NGAL indicates that the individual is either at increases risk for developing or has a malignant or metastatic cancer.

13. The method of claim 1 or 12, wherein the free NGAL comprises monomeric NGAL.

14. The method of claim 12, wherein the control level is a level of free NGAL associated with a healthy individual.

15. A method for monitoring cancer or risk of developing cancer in an individual, comprising the steps of:
a) determining a level of free NGAL in a first test urine sample obtained from an individual to obtain a first level of free NGAL;
b) determining a level of free NGAL in a second urine sample obtained from the individual to obtain a second level of free NGAL; and
c) comparing the first and second levels of free NGAL;
wherein the cancer is a cancer of epithelial origin.

16. The method of claim 16, wherein when the second level is greater than the first level, the individual is at greater risk of having o developing cancer or is at greater risk of having or developing a metastasizing cancer.

17. The method of claim 15, wherein the individual is a post-cancer treatment patient, and when the second level is greater than the first level, the post-cancer treatment patient is referred for treatment of recurrence of the cancer.

18. A method for monitoring for cancer recurrence in a post-cancer treatment patient, comprising the steps of:
a) determining a level of free NGAL in a first test urine sample obtained from a post-cancer treatment patient to obtain a first level of free NGAL;
b) determining a level of free NGAL in a second urine sample obtained from the post-cancer treatment patient to obtain a second level of free NGAL; and
c) comparing the first and second levels of free NGAL, wherein when the second level is greater than the first level, the post-cancer treatment patient is referred for treatment of recurrence of the cancer;
wherein the cancer is a cancer of epithelial origin.

## Patentansprüche

1. Verfahren zur Bestimmung, ob ein Individuum ein Risiko für die Entwicklung von Krebs aufweist oder Krebs entwickelt hat, umfassend die folgenden Schritte:
a) Bestimmung eines Spiegels von freiem NGAL in einer von einem Individuum gewonnenen Testurinprobe;
b) Vergleich des Spiegels von freiem NGAL in der Testurinprobe mit einem Kontrollspiegel von freiem NGAL;
wobei ein Spiegel von freiem NGAL in der Testprobe höher als ein Kontrollspiegel von freiem NGAL anzeigt, dass das Individuum entweder ein erhöhtes Risiko für die Entwicklung von Krebs aufweist, oder an Krebs leidet;
wobei der Krebs ein Krebs epithelialen Ursprungs ist.

2. Verfahren nach Anspruch 1, wobei der Krebs epithelialen Ursprungs ausgewählt ist aus der Gruppe, bestehend aus Brustkrebs, einem Basalzellkarzinom, einem Adenokarzinom, Gastrointestinalkrebs, Lippenkrebs, Mundkrebs, Ösophaguskrebs, Dünndarmkrebs, Magenkrebs, Kolonkrebs, Leberkrebs, Blasenkrebs, Pankreaskrebs, Ovarialkrebs, Cervixkrebs, Lungenkrebs, Hautkrebs, Nierenkrebs, Prostatakrebs und einem Nierenzellkarzinom.

3. Verfahren nach Anspruch 1, wobei der Krebs Brust- und/oder Ovarialkrebs ist.

4. Verfahren nach Anspruch 1, wobei der Spiegel von freiem NGAL mittels eines Verfahrens gemessen wird, umfassend die folgenden Schritte:
a) Inkontaktbringen der Testprobe oder einer Aufbereitung davon, mit einem Antikörper-basierten Bindungsteil, der zur Bildung eines Antikörper-NGAL-Komplexes bevorzugt an freies NGAL bindet; und
b) Nachweis des Vorliegens des Antikörper-NGAL-Komplexes, wobei so der Spiegel von vorliegendem freiem NGAL gemessen wird.

5. Verfahren nach Anspruch 1, wobei der Antikörper-basierte Bindungsteil mit einer nachweisbaren Markierung markiert ist.

6. Verfahren nach Anspruch 1, wobei die Markierung aus der Gruppe ausgewählt ist, bestehend aus einer radioaktiven Markierung, einer Haptenmarkierung, einer Fluoreszenzmarkierung und einer Enzymmarkierung.

7. Verfahren nach Anspruch 1, wobei der Antikörper-basierte Bindungsteil ein Antikörper ist.

8. Verfahren nach Anspruch 1, wobei der Antikörper ein monoklonaler Antikörper ist.

9. Verfahren nach Anspruch 1, ferner umfassend den Schritt des Überprüfens der Ergebnisse, und wenn der Urin einen höheren Spiegel von freiem NGAL aufweist als der Kontrollspiegel von freiem NGAL, Überweisung des Individuums zum weiteren Testen auf Krebs.

10. Verfahren nach Anspruch 1, wobei der Spiegel von freiem NGAL mittels massenspektrometrischer Verfahren gemessen wird.

11. Verfahren nach Anspruch 1, wobei das Individuum ein Patient nach einer Krebsbehandlung ist, und wenn ein Spiegel von freiem NGAL in der Testprobe höher ist als ein Kontrollspiegel von freiem NGAL, der Patient nach einer Krebsbehandlung zur Behandlung eines Rezidivs des Krebses überwiesen wird.

12. Verfahren zum Staging eines Krebses epithelialen Ursprungs, umfassend die folgenden Schritte:
a) Bestimmung eines Spiegels von freiem NGAL in einer von einem Individuum gewonnenen Testurinprobe;
b) Vergleich des Spiegels von freiem NGAL in der Testurinprobe mit einem Kontrollspiegel von freiem NGAL;
wobei ein Spiegel von freiem NGAL in der Testprobe höher als ein Kontrollspiegel von freiem NGAL anzeigt, dass das Individuum entweder ein erhöhtes Risiko für die Entwicklung eines malignen oder metastatischen Krebses aufweist oder an einem malignen oder metastatischen Krebs leidet.

13. Verfahren nach Anspruch 1 oder 12, wobei das freie NGAL monomeres NGAL umfasst.

14. Verfahren nach Anspruch 12, wobei der Kontrollspiegel ein Spiegel von freiem NGAL ist, der mit einem gesunden Individuum assoziiert wird.

15. Verfahren zur Überwachung von Krebs oder eines Risikos für die Entwicklung von Krebs bei einem Individuum, umfassend die folgenden Schritte:
a) Bestimmung eines Spiegels von freiem NGAL in einer ersten Testurinprobe, die von einem Individuum zum Erhalt eines ersten Spiegels von freiem NGAL gewonnen wird;
b) Bestimmung eines Spiegels von freiem NGAL in einer zweiten Urinprobe, die von dem Individuum zum Erhalt eines zweiten Spiegels von freiem NGAL gewonnen wird; und
c) Vergleich der ersten und zweiten Spiegel von freiem NGAL;
wobei der Krebs ein Krebs epithelialen Ursprungs ist.

16. Verfahren nach Anspruch 16, wobei, wenn der zweite Spiegel höher als der erste Spiegel ist, das Individuum ein größeres Risiko, an Krebs zu leiden oder für die Entwicklung eines Krebses aufweist oder ein größeres Risiko, an metastasierendem Krebs zu leiden oder für die Entwicklung eines metastasierenden Krebses aufweist.

17. Verfahren nach Anspruch 15, wobei das Individuum ein Patient nach der Krebsbehandlung ist, und wenn der zweite Spiegel höher als der erste Spiegel ist, der Patient nach der Krebsbehandlung zur Behandlung eines Rezidivs des Krebses überwiesen wird.

18. Verfahren zur Überwachung auf ein Krebsrezidiv bei einem Patienten nach der Krebsbehandlung, umfassend die folgenden Schritte:
a) Bestimmung eines Spiegels von freiem NGAL in einer ersten Testurinprobe, die von einem Patienten nach der Krebsbehandlung zum Erhalt eines ersten Spiegels von freiem NGAL gewonnen wird;
b) Bestimmung eines Spiegels von freiem NGAL in einer zweiten Urinprobe, die von dem Patienten nach der Krebsbehandlung zum Erhalt eines zweiten Spiegels von freiem NGAL gewonnen wird; und
c) Vergleich des ersten und des zweiten Spiegels von freiem NGAL, wobei, wenn der zweite Spiegel höher als der erste Spiegel ist, der Patient nach der Krebsbehandlung zur Behandlung eines Rezidivs des Krebses überwiesen wird;
wobei der Krebs ein Krebs epithelialen Ursprungs ist.

## Revendications

1. Procédé pour déterminer si un individu est à risque de développer ou a développé un cancer, comprenant les étapes consistant à:
a) déterminer un taux de NGAL libre dans un échantillon d'urine de test obtenu d'un individu;
b) comparer le taux de NGAL libre dans l'échantillon d'urine de test à un taux témoin de NGAL libre;
dans lequel un taux de NGAL libre dans l'échantillon de test plus élevé qu'un taux témoin de NGAL libre indique que l'individu est soit à risque accru de développer un cancer soit a un cancer;
où le cancer est un cancer d'origine épithéliale.

2. Procédé selon la revendication 1, dans lequel le cancer d'origine épithéliale est sélectionné parmi le groupe consistant en cancer du sein, carcinome à cellules basales, adénocarcinome, cancer gastro-intestinal, cancer des lèvres, cancer de la bouche, cancer de l'oesophage, cancer de l'intestin grêle, cancer de l'estomac, cancer du côlon, cancer du foie, cancer de la vessie, cancer du pancréas, cancer de l'ovaire, cancer du col de l'utérus, cancer du poumon, cancer de la peau, cancer du rein, cancer de la prostate et carcinome à cellules rénales.

3. Procédé selon la revendication 1, dans lequel le cancer est un cancer du sein et/ou de l'ovaire.

4. Procédé selon la revendication 1, dans lequel le taux de NGAL libre est mesuré par un procédé comprenant les étapes consistant à:
a) mettre l'échantillon de test, ou une préparation du même, en contact avec une fraction de liaison à base d'anticorps qui se lie préférentiellement à une NGAL libre pour former un complexe d'anticorps-NGAL; et
b) détecter la présence du complexe d'anticorps-NGAL, mesurant ainsi le taux de NGAL libre présent.

5. Procédé selon la revendication 1, dans lequel la fraction de liaison à base d'anticorps est marquée avec un marqueur détectable.

6. Procédé selon la revendication 1, dans lequel le marqueur est sélectionné parmi le groupe consistant en un marqueur radioactif, un marqueur haptène, un marqueur fluorescent et un marqueur enzymatique.

7. Procédé selon la revendication 1, dans lequel la fraction de liaison à base d'anticorps est un anticorps.

8. Procédé selon la revendication 1, dans lequel l'anticorps est un anticorps monoclonal.

9. Procédé selon la revendication 1, comprenant en outre l'étape consistant à revoir les résultats et, si l'urine présente un taux de NGAL libre plus élevé que le taux témoin de NGAL libre, référer l'individu pour d'autres tests pour le cancer.

10. Procédé selon la revendication 1, dans lequel le taux de NGAL libre est mesuré par des méthodes de spectrophotométrie de masse.

11. Procédé selon la revendication 1, dans lequel l'individu est un patient ayant suivi un traitement anticancéreux et lorsqu'un taux de NGAL libre dans l'échantillon de test est plus élevé qu'un taux témoin de NGAL libre, le patient ayant suivi un traitement anticancéreux est référé pour traitement de récurrence du cancer.

12. Procédé de détermination du stade d'un cancer d'origine épithéliale, comprenant les étapes consistant à:
a) déterminer un taux de NGAL libre dans un échantillon d'urine de test obtenu d'un individu;
b) comparer le taux de NGAL libre dans l'échantillon d'urine de test à un taux témoin de NGAL libre;
où un taux de NGAL libre dans l'échantillon de test plus élevé qu'un taux témoin de NGAL libre indique que l'individu est soit à risque accru de développer ou a un cancer malin ou métastatique.

13. Procédé selon la revendication 1 ou 12, dans lequel la NGAL libre comprend de la NGAL monomérique.

14. Procédé selon la revendication 12, dans lequel le taux témoin est un taux de NGAL libre associé à un individu sain.

15. Procédé de surveillance d'un cancer ou d'un risque de développer un cancer chez un individu, comprenant les étapes consistant à:
a) déterminer un taux de NGAL libre dans un premier échantillon d'urine de test obtenu d'un individu, afin d'obtenir un premier taux de NGAL libre;
b) déterminer un taux de NGAL libre dans un deuxième échantillon d'urine obtenu de l'individu, pour obtenir un deuxième taux de NGAL libre; et
c) comparer le premier et le deuxième taux de NGAL libre;
où le cancer est un cancer d'origine épithéliale.

16. Procédé selon la revendication 16, dans lequel, lorsque le deuxième taux est plus élevé que le premier taux, l'individu est plus à risque d'avoir ou de développer un cancer ou est plus à risque d'avoir ou de développer un cancer qui se métastase.

17. Procédé selon la revendication 15, dans lequel l'individu est un patient ayant suivi un traitement anticancéreux et lorsque le deuxième taux est plus élevé que le premier taux, le patient ayant suivi un traitement anticancéreux est référé pour traitement de récurrence du cancer.

18. Procédé de surveillance pour une récurrence du cancer chez un patient ayant suivi un traitement anticancéreux, comprenant les étapes consistant à:
a) déterminer un taux de NGAL libre dans un premier échantillon d'urine de test obtenu d'un patient ayant suivi un traitement anticancéreux afin d'obtenir un premier taux de NGAL libre;
b) déterminer un taux de NGAL libre dans un deuxième échantillon d'urine obtenu du patient ayant suivi un traitement anticancéreux pour obtenir un deuxième taux de NGAL libre; et
c) comparer le premier et le deuxième taux de NGAL libre, où lorsque le deuxième taux est plus élevé que le premier taux, le patient ayant suivi un traitement anticancéreux est référé pour traitement de récurrence du cancer;
où le cancer est un cancer d'origine épithéliale.
